# EUROPÄISCHE PATENTANMELDUNG

(11) **EP 4 516 428 A1**
(43) Veröffentlichungstag der Anmeldung: **05.03.2025**
(21) Anmeldenummer: 23194529.6
(22) Anmeldetag: 31.08.2023
(51) Int. Cl.: B22F 7/06, B22F 10/28, A61F 2/30, B33Y 10/00, B33Y 80/00, A61F 2/02

(54) **VERFAHREN ZUR HERSTELLUNG EINES IMPLANTATS SOWIE IMPLANTAT**

(71) Anmelder: Fraunhofer-Gesellschaft zur Förderung der angewandten Forschung e.V., 80686 München (DE)
(72) Erfinder: Horn, Max, 80333 München (DE); Seidel, Christian, 85748 Garching (DE); Schlick, Georg, 86156 Augsburg (DE)
(74) Vertreter: Pfenning, Meinig & Partner mbB

(57) **Zusammenfassung**

Die Erfindung bezieht sich auf ein Verfahren zur Herstellung eines Implantats (1), wobei das Implantat wenigstens zwei miteinander verbundene Bauteilabschnitte (1a, 1b, 1c) aufweist, die wenigstens in einem Bereich (1d), in dem sie aneinander angrenzen, durch ein additives Fertigungsverfahren in Form eines Pulverbett-Verbindungsprozesses hergestellt werden, wobei ein erster Bauteilabschnitt (1a) aus einem ersten Material und ein zweiter Bauteilabschnitt (1b) aus einem zweiten Material hergestellt wird, wobei das erste und das zweite Material sich durch das Maß ihrer Bio-Kompatibilität unterscheiden. Das additive Herstellungsverfahren ermöglicht dabei eine sehr freie Gestaltung der verschiedenen Bauteilabschnitte des Implantats.

## Beschreibung

Die Erfindung liegt auf dem Gebiet der Werkstoff- und Prozesstechnik und befasst sich mit additiven Fertigungsverfahren. Weiter bezieht sich die Erfindung auf Implantate, das heißt auf Bauteile, die in der Medizintechnik Anwendung finden.

Implantate sind für viele verschiedene Anwendungsfälle der Medizin bekannt. Teilweise können Implantate als Aggregate mit einer Funktion gestaltet sein, die in irgendeiner Form in den Körper eines Patienten integriert werden sollen, weil sie dort ihre Funktion ausüben, ohne dass es genau auf die Position ankommt. Hierzu zählen z.B. Herzschrittmacher oder Defibrillatoren oder Bauteile, die Substanzen abgeben, die im Körper des Patienten eine Wirkung ausüben. Andererseits sind Implantate bekannt, die eine definierte mechanische Funktion in Verbindung mit dem Halte- oder Bewegungssystem eines Patienten erfüllen und bei denen die Positionierung eine größere Rolle spielt. Zu den letztgenannten Implantaten gehören beispielsweise die Endoprothesen zur Knochen- oder Gelenksubtitution. Diese sollen in vielen Fällen unter anderem mit Knochengewebe und/oder Weichteilgewebe zusammenwachsen. Um derartige Aufgaben zu erfüllen, sind verschiedene Materialien und Implantatformen (z. B. Autotransplantate oder Allotransplantate) entwickelt worden. Der erfolgreiche Einsatz von Implantaten hängt dabei wesentlich von der Interaktion von Zellen oder lebendem Gewebe mit dem Implantat-Material ab. Das gewünschte Anheften von Knochenzellen an Implantate und die damit verbundene Knochenbildung kann mit dreidimensionalen (3D) Gitter- oder Gerüststrukturen (engl. Scaffold Structures) gefördert werden.

Materialien für die Verwendung bei der Herstellung von Implantaten müssen bestimmte Anforderungen erfüllen. Die biologische Verträglichkeit und die mechanischen Eigenschaften dieser Materialien gelten als zwei der kritischen Parameter, die ihre Eignung für die jeweilige Anwendung bestimmen. Außerdem sind die Umgebungsbedingungen im menschlichen Körper stark korrosiv. Implantate sollen deshalb auch eine langfristige Korrosionsbeständigkeit aufweisen.

Hinsichtlich der mechanischen Eigenschaften sollten Implantate an das oder die Wirtsgewebe angepasst sein. Im Falle von zu geringer Steifigkeit und Festigkeit versagt das Implantat bei Belastung und erfüllt seinen Zweck nicht. Insbesondere im Falle von zu hoher Steifigkeit, kann es zum sogenannten Stress Shielding kommen. Der höhere Elastizitätsmodul von häufig verwendeten Implantat-Materialien kann zu unerwünschtem Knochenabbau führen, da der Knochen nicht ausreichend belastet wird, was wiederum das Ergebnis einer Reduzierung der vom Implantat auf den Knochen übertragenen Spannung ist. Eine ausreichende Belastung ist somit für den Knochenumbau notwendig, um die um die Knochenstruktur ausreichend an die äußeren Belastungen anzupassen. Zu geringe Belastung führt damit zum Knochenabbau, was häufig in Lockerungen der Implantate und Knochenfrakturen resultiert. Zum Vergleich der Größen sollen folgende Werte dienen: Das Elastizitätsmodul eines menschlichen Skelettknochens wird in der Literatur mit 12-20 GPa angegeben, wobei dieser maßgeblich aus der Kortikalis, der massiven äußeren Knochenschicht, bestimmt wird, während der Elastizitätsmodul von CoCr-Legierungen bei 230 GPa, der von rostfreiem Stahl (316L bzw. 1.4404) bei 210 GPa und der von Titan bei ca. 110 GPa liegt (Ryan et al. 2006).

Da in vielen Fällen kein anderes Material außer dem tatsächlichen Knochen im Falle von Osseo- Implantaten oder auch bei Weichteil- Implantaten die notwendigen Eigenschaften gleichzeitig erfüllt, ist es besonders für LangzeitImplantate sinnvoll, Bauteileigenschaften anforderungsspezifisch anzupassen. Beispielsweise ist eine Variation der Bauteilporosität, von dichtem Vollmaterial hin zu offenporigen Scaffold-Strukturen mit variierender Porosität im Zusammenhang mit Knochenimplantaten bereits grundsätzlich bekannt. Außerdem werden mithilfe konventioneller Fertigungsverfahren bereits Implantate aus unterschiedlichen Werkstoffen zusammengesetzt. Dabei werden die einzelnen Werkstoffe allerdings bisher separat verarbeitet und am Ende gefügt bzw. verbunden. Das bringt sehr aufwändige und komplizierte Prozessketten mit sich.

Der vorliegenden Erfindung liegt vor dem Hintergrund des Standes der Technik die Aufgabe zugrunde, ein Verfahren zur Herstellung eines Implantats sowie ein Implantat zu schaffen, das eine Anpassung an komplexe Anforderungen erlaubt.

Die Aufgabe wird mit den Merkmalen der Erfindung gemäß den unabhängigen Ansprüchen durch ein Herstellungsverfahren sowie durch ein mit dem Verfahren hergestelltes Implantat gelöst. Die abhängigen Ansprüche stellen mögliche Ausgestaltungen der Lösung vor.

Demgemäß bezieht sich die Erfindung auf ein Verfahren zur Herstellung eines Implantats, wobei das Implantat wenigstens zwei miteinander verbundene Bauteilabschnitte aufweist, die wenigstens in einem Bereich, in dem sie aneinander angrenzen, durch ein additives Fertigungsverfahren in Form eines Pulverbett-Verbindungsprozesses hergestellt werden, wobei ein erster Bauteilabschnitt aus einem ersten Material und ein zweiter Bauteilabschnitt aus einem zweiten Material hergestellt wird, wobei das erste und das zweite Material sich durch das Maß ihrer Bio-Kompatibilität unterscheiden.

Unter einem Pulverbett- Verbindungsprozess soll in diesem Zusammenhang ein additiver Fertigungsprozess verstanden werden, bei dem ein oder mehrere Werkstoffe in Form eines Pulvermaterials schichtweise auf eine Bauteilplattform oder ein Bauteil aufgetragen und darauf selektiv verfestigt wird/werden. Dabei kann die Verfestigung auf eine von mehreren bekannten Arten stattfinden, beispielsweise durch ein Binder-Jet- Verfahren, ein Sinterverfahren oder ein Schmelzverfahren, wobei im Sinter- und Schmelzverfahren jeweils ein gezielter lokaler Energieeintrag in das aufgetragene Pulver erfolgt, um dieses lokal und selektiv zu verfestigen. Ein in diesem Zusammenhang bekannter Prozess ist beispielsweise das Pulverbett- Laserschmelzverfahren, auf das das erfindungsgemäße Verfahren jedoch nicht eingeschränkt werden soll, ebenso wie auch alle bekannten Arten von Pulvern, z.B. Pulver mit vorwiegend runden Partikeln und Pulver mit eher unregelmäßig geformten Partikeln, verwendbar sind.

Das Implantat kann im Ganzen oder auch teilweise in einem additiven Fertigungsverfahren hergestellt werden. Zur teilweisen Herstellung in einem additiven Fertigungsverfahren können beispielsweise vorgefertigte Teile in einem additiven Fertigungsverfahren mit Pulverschichten bedeckt werden, die danach verfestigt werden. Allerdings soll das Implantat in einem Bereich, in dem die verschiedenen Bauteilabschnitte aneinander angrenzen, in dem additiven Herstellungsverfahren aufgebaut werden, um in diesem Bereich die Vorteile dieses Herstellungsverfahren nutzen zu können. Dadurch können Bauteilabschnitte auch aus verschiedenen Materialien in einfacher und zuverlässiger Form zusammengefügt werden, wobei das additive Fertigungsverfahren ein höchstes Maß an Gestaltungsmöglichkeiten bietet, sowohl bezüglich der geometrischen Gestaltung, Größe, Lage und Orientierung des Grenzbereichs, als auch bezüglich der Materialzusammensetzung in diesem Bereich. Besonders durch die Zusammenstellung des Implantats aus zwei Materialien, die ein verschiedenes Maß an Bio- Kompatibilität bieten, kann das Implantat individuell angepasst werden, beispielsweise, indem ein erster Teil, des Implantats, der in einen ersten Bereich eines Körpers eingebracht werden soll, ein erstes Maß an Biokompatibilität bietet, während ein zweiter Teil des Implantats, der in einen zweiten Bereich des Körpers hineinragt, eine davon verschiedene Biokompatibilität, insbesondere ein davon verschiedenes Maß an Biokompatibilität aufweist. Das Implantat kann in diesem Zusammenhang in einem ersten Bauteilabschnitt, in dem die Anforderungen an die Biokompatibilität anders sind als in dem zweiten Bauteilabschnitt, beispielweise aus einem Material bestehen, das andere Anforderungen besser erfüllt als das Material des zweiten Bauteilabschnitts, beispielsweise Anforderungen an die Materialsteifigkeit, Elastizität oder Ermüdungsfestigkeit. Eine mögliche Realisierung eines Implantats aus zwei Materialien verschiedener Biokompatibilität kann in vielen Fällen dadurch gegeben sein, dass das erste Material ein Titanmaterial, das heißt Titan oder eine Legierung mit Titan als Hauptbestandteil, und das zweite Material ein Tantalmaterial, das heißt Tantal oder eine Legierung mit dem Hauptbestandteil Tantal, ist. Hierauf wird im Folgenden noch detaillierter eingegangen.

Grundsätzlich sind mit Implantaten im Rahmen dieses Textes alle möglichen Arten von Implantaten gemeint, insbesondere sowohl inaktive als auch aktive Implantate, die stoffwechselwirksame Stoffe abgeben, beispielsweise aus einer porösen Struktur.

In diesem Zusammenhang kann beispielsweise auch vorgesehen sein, dass das erste und das zweite Material sich durch das Maß ihrer Bio-Inertheit unterscheiden.

Eine mögliche Definition der Biokompatibilät ist im Rahmen der Erfindung beispielsweise dadurch gegeben, dass Materialien und Werkstoffe, die im direkten Kontakt mit lebenden Geweben stehen, keinen negativen Einfluss auf deren Stoffwechsel ausüben. Je geringer also nach dieser Definition ein negativer Einfluss oder je größer ein positiver Einfluß eines Materials oder Werkstoffes auf den Stoffwechsel von lebenden Geweben ist, mit denen das Material oder der Werkstoff im Kontakt steht, umso höher ist die Biokompatibilität dieses Materials. Unter einem negativen Einfluss kann dabei jeder Einfluss auf den Stoffwechsel verstanden werden, der den Stoffwechsel derart beeinflusst, dass physiologisch unerwünschte Prozesse in Gang gesetzt oder unterstützt werden. Zwei Werkstoffe oder Materialien, die eine verschiedene Biokompatibilität aufweisen, können sich auch lediglich in bestimmten Aspekten der Biokompatibilität unterscheiden, indem beispielsweise das eine Material kompatibler mit einer ersten Gewebeart (z.B. Weichteilgewebe) und das andere Material kompatibler mit einer zweiten Gewebeart (z.B. Knochengewebe) ist.

Als weitere mögliche Definition des Gegenteils oder der Abwesenheit von Biokompatibilität ergibt sich aus der Formulierung einer Norm die "Wahrscheinlichkeit von gesundheitlichen Schäden aufgrund des Medizinprodukts oder Wechselwirkungen mit den Materialien". Die Biokompatibilität lässt sich dann bemessen als "Wahrscheinlichkeit der Freiheit oder Abwesenheit von gesundheitlichen Schäden aufgrund des Medizinprodukts oder Wechselwirkungen mit den Materialien".

Für Knochenimplantate, das heißt Implantate, die zum Zusammenwachsen mit Knochen eines Körpers vorgesehen sind, kann die Biokompatibilität auch beispielsweise als Fähigkeit zur Osseointegration bei einer definierten Oberflächenbeschaffenheit des Implantats definiert sein. Wenn im Rahmen dieses Textes von verschiedener Biokompatibilität von Materialien gesprochen wird, so ist dabei die Biokompatibilität bei jeweils gleicher Oberflächenbeschaffenheit gemeint.

Bei der Biokompatibilität kann auch zwischen Strukturverträglichkeit und Oberflächenverträglichkeit unterschieden werden. Um strukturelle Kompatibilität zu erreichen, müssen die Struktur und die mechanischen Eigenschaften des Implantats an die des Wirtsgewebes angepasst sein. In ähnlicher Weise müssen die Oberflächeneigenschaften der Implantate (chemisch, biologisch und morphologisch) an die des Wirtsgewebes angepasst werden. In diesem Fall ist eine klinisch gewünschte Wechselwirkung zwischen dem Wirtsgewebe und der Oberfläche des Implantats angestrebt, um eine stabile Implantatverankerung zu erreichen. Dabei muss die Oberfläche auch an die angestrebte Verweildauer im Patienten angepasst werden. Für Kurzzeitimplantate eignet sich insbesondere eine glatte Oberfläche, damit das Implantat nach abgeschlossener Heilung wieder entfernt werden kann. Für Langzeitimplantate ist jedoch eine strukturierte Oberfläche für das An- und/oder Einwachsen von Knochengewebe besser geeignet, um eine langfristige, stabile Verankerung des Implantats zu ermöglichen (Bram 2013). Typische Beispiele sind Gelenkimplantate. Das Einwachsen von Knochen (d. h. die Osseointegration) und deren Vaskularisierung können durch eine definierte, offene und vernetzte Porosität des Implantats bei geeigneter Materialwahl gefördert werden. Die Adhäsion von Zellen, die für das Anwachsen von Knochengewebe grundlegend ist, kann außerdem durch eine definierte Mikro-Oberflächenrauheit der Scaffold-Strukturen gefördert werden.

Die Bio- Inertheit von Werkstoffen und Materialien kann allgemein als die Eigenschaft definiert werden, keine irgendwie geartete Wechselwirkung oder Reaktion des mit ihnen im direkten Kontakt befindlichen lebenden Gewebes hervorzurufen.

Es kann bei der Zusammensetzung des Implantats in einer Realisierung beispielsweise auch vorgesehen sein, dass ein erster der Bauteilabschnitte in massiver Form aus dem ersten Material und ein zweiter Bauteilabschnitt in poröser, schwammartiger Form aus dem zweiten Material gefertigt wird.

Eine Ausgestaltung des beschriebenen Verfahrens kann vorsehen, dass das erste und/oder das zweite Material ein Metall ist. Viele Metalle eignen sich gut zur Implantation und weisen bezüglich der Möglichkeiten der Formgebung, der Verarbeitung, der Festigkeit, des Elastizitätsmoduls und der Stabilität gute Eigenschaften auf, wobei durch die Auswahlmöglichkeiten unter verschiedenen Metallen ein breites Spektrum von mechanischen Eigenschaften gestaltbar ist.

Eine weitere Ausgestaltung kann vorsehen, dass das erste oder das zweite Material Tantal oder eine Tantallegierung ist.

Tantal sowie Tantallegierungen weisen eine höhere Bio-aktivität und Biokompatibilität auf als viele andere Metalle, beispielsweise Titan oder seine Legierungen. Somit eignet sich Tantal gut für einen Teil eines Implantats, der sich mit lebenden Gewebe durch Einwachsen verbinden soll.

Es kann weiter vorgesehen sein, dass das erste oder das zweite Material Titan oder eine Titanlegierung, insbesondere die Titanlegierung Ti6Al4V ist.

Titan und seine Legierungen weisen eine größere Bio- Inertheit auf als viele andere Metalle, beispielsweise eine größere Bio- Inertheit als Tantal und seine Legierungen, das heißt, es ruft im Kontakt mit lebendem Gewebe weniger oder geringere Reaktionen, insbesondere weniger oder geringere Stoffwechselveränderungen hervor.

Zudem verfügt Titan über besser geeignete mechanische Eigenschaften, beispielsweise eine höhere mechanische Festigkeit als Tantal, während Tantal eine höhere Osseointegrationsleistung aufweist als Titan.

Zur Verarbeitung von Tantal, Titan sowie Titanlegierungen jeweils für sich zu homogenen Bauteilen sind bereits Pulverbett- Verbindungsverfahren bekannt geworden. Vereinzelt gibt es auch Ansätze zur kombinierten Verarbeitung von Tantal und einer Titanlegierung zu einem Bauteil in konventionellen Fügeverfahren. Jedoch ist die Verarbeitung in einem additiven Fertigungsverfahren zu einem Implantat mit der gezielten Nutzung der verschiedenen Biokompatibilitäten nicht bekannt.

Es kann weiter bei einem Verfahren der oben beschriebenen Art vorgesehen sein, dass zwischen dem ersten Bauteilabschnitt und dem zweiten Bauteilabschnitt ein Verbindungsabschnitt gebildet wird, der aus einer Mischung wenigstens des ersten Materials und des zweiten Materials, insbesondere, falls sowohl das erste als auch das zweite Material ein Metall ist, wenigstens teilweise aus einer Legierung mit dem ersten und dem zweiten Material besteht.

Das Vorhandensein einer Mischung verschiedener Materialien soll dabei bedeuten, dass in dem Verbindungsabschnitt jeweils auch Raumbereiche oder Teilchen des einen Materials in mehreren Raumrichtungen zwischen Raumbereichen oder Teilchen des jeweils anderen Materials eingebettet sind, so dass es auf einer mikroskopischen Ebene nicht möglich ist, eine klare Trennlinie/Trennfläche zwischen den beiden Materialien zu bestimmen, derart dass auf jeder Seite der Trennlinie ausschließlich Teilchen oder Raumbereiche eines einzigen der Materialien liegen.

In einer weiteren Implementierung des Verfahrens kann vorgesehen sein, dass der Verbindungsabschnitt mit einer gradierten Zusammensetzung gebildet wird, die in einer oder mehreren Richtungen ortsabhängig variierende Anteile des ersten und des zweiten Materials aufweist.

Diese Anteile der Materialien können beispielsweise Massen- oder Volumenanteile sein. Die Mischung kann auf einer Größenebene vorgesehen sein, die kleine Raumbereiche jedes der Materialien vorsieht, die beispielsweise monokristallin oder zumindest homogen ausgebildet sind. Die Mischung kann jedoch auch als Legierung auf atomarer oder molekularer Ebene vorliegen.

Es kann auch vorgesehen sein, dass der Mischung des ersten und zweiten Materials im Verbindungsabschnitt ein drittes Material in Pulverform vor der Verfestigung beigegeben wird.

Es kann weiter bei dem beschriebenen Verfahren vorgesehen sein, dass der erste Bauteilabschnitt und der zweite Bauteilabschnitt zur Verfestigung jeweils mit einem Energiestrahl bestrahlt wird, wobei durch die Wahl unterschiedlicher Strahlparameter und/oder Strahlführungsparameter in den beiden Bauteilabschnitten verschiedene lokale Verbindungstemperaturen erreicht werden. Es kann weiter vorgesehen sein, dass der Energiestrahl zum Verfestigen der Materialien als Laserstrahl oder als Elektronenstrahl ausgebildet ist.

Unter den Strahlparametern können dabei beispielsweise die Energieintensität, Leistung, die Leistungsdichte, der Strahldurchmesser oder ähnliche Parameter verstanden werden, sowie bei einem Laserstrahl der duty cycle, die Wellenlänge, Modulation. Als Strahlführungsparameter kann beispielsweise die Geschwindigkeit bezeichnet werden, mit der ein Strahl über das Target bewegt wird und die Geometrie des Wegs, den der Strahl über das Target zurücklegt.

Dabei kann sich ergeben, dass in dem Verbindungsabschnitt durch die Wahl der Strahlparameter und/oder Strahlführungsparameter eine Verbindungstemperatur erreicht wird, die zwischen den Verbindungstemperaturen des ersten und des zweiten Bauteilabschnitts oder die oberhalb der höheren der Verbindungstemperaturen des ersten und des zweiten Bauteilabschnitts liegt. Wird eine Verbindungstemperatur erreicht, die oberhalb der höheren der Verbindungstemperaturen des ersten und des zweiten Bauteilabschnitts liegt, so wird dadurch das Aufschmelzen der beiden Materialien im Verbindungsabschnitt besonders gefördert, so dass eine intensive Vermischung der beiden Materialien erreicht werden kann.

Beim Auftrag der Schichten in dem beschriebenen Verfahren kann vorgesehen sein, dass in dem Verbindungsabschnitt jeweils in einem Schritt ausschließlich Pulvermengen abgelegt werden, die entweder aus dem ersten Material oder aus dem zweiten Material oder aus einem dritten Material bestehen oder dass in einem Schritt zumindest bereichsweise im Verbindungsbereich eine Pulvermischung abgelegt wird, die das erste und das zweite Material oder das erste und das zweite Material sowie ein drittes Material enthält.

Werden jeweils in einem Schritt ausschließlich Pulvermengen abgelegt, die entweder aus dem ersten Material oder aus dem zweiten Material bestehen, so findet eine Vermischung oder Legierung erst unter dem Einfluss des Energieeintrags durch eine Bestrahlung statt. Der Aufwand für den Pulverauftrag wird dabei minimiert, da kein Pulver gemischt und keine gesonderte Misch- oder Zuführungseinrichtung bereitgestellt werden muss. Es kann allerdings in manchen Fällen sinnvoll sein, die Schichtdicke des Pulverauftrags zumindest im Verbindungsbereich zu variieren.

Zusätzlich können auch noch, zumindest im Verbindungsbereich, Schichten mit einem weiteren, dritten Material zwischen den Schichten des ersten und des zweiten Materials hinzugefügt werden. Dieses dritte Material kann sich beispielsweise auf das Schmelzverhalten oder die in der Legierung realisierten Materialeigenschaften auswirken.

Wenn zumindest bereichsweise im Verbindungsbereich eine Pulvermischung abgelegt wird, die das erste und das zweite Material enthält, so kann durch vorherige Durchmischung der Materialien eine homogenere Mischung der Bestandteile erreicht werden. Die Pulvermischung kann im Verbindungsbereich optional auch noch Zusätze eines dritten Materials enthalten, das beispielsweise die Durchmischung des ersten und zweiten Materials verbessern, den Verarbeitungsprozess erleichtern oder nach der Verfestigung die Eigenschaften der Mischung oder Legierung des ersten und zweiten Materials durch den Zusatz des dritten Materials verbessern kann. Das dritte Material kann auch separat in einer Schicht abgelegt werden, die jeweils anderen Schichten aus einer Pulvermischung des ersten und zweiten Materials benachbart ist. Es ist zudem auch möglich, im Verbindungsbereich Pulvermischungen abzulegen, die nur das erste und das dritte oder nur das zweite und das dritte Material enthalten.

Bei dem beschriebenen Verfahren kann weiter vorgesehen sein, dass jede Pulverschicht des ersten und zweiten Materials jeweils beide Bauteilabschnitte und insbesondere den Verbindungsabschnitt vollflächig abdeckend abgelegt wird und dass nicht verfestigte Bereiche des aufgetragenen Materials nach der Verfestigung von Teilen der jeweiligen Schicht abgetragen werden.

Es kann andererseits jedoch auch vorgesehen sein, dass eine oder mehrere oder alle Pulverschichten des ersten und zweiten Materials jeweils nur in den Bereichen der Bauteilabschnitte und des Verbindungsabschnitts abgelegt werden, an denen sie verfestigt werden.

Eine weitere Ausgestaltung des Verfahrens kann vorsehen, dass wenigstens ein Verbindungsbereich sich durch wenigstens zwei, insbesondere wenigstens drei, weiter insbesondere wenigstens zehn Schichten erstreckt, wobei weiter insbesondere wenigstens eine Grenze des Verbindungsbereiches schräg durch die Schichten und/oder schräg zur z- Richtung des Auftragsverfahrens verläuft und/oder dass der erste und/oder der zweite Bauteilabschnitt und/oder der Verbindungsabschnitt einen oder mehrere Hohlräume aufweist und insbesondere eine schwammartige Struktur aufweist.

Damit ergibt sich mit dem Verfahren die Möglichkeit, Anteile von zwei oder mehr Materialien in dem herzustellenden Implantat in jeder Raumrichtung, insbesondere in der Richtung des Übergangs vom ersten zum zweiten Bauteilabschnitt, gradiert zu dosieren, so dass die Zusammensetzung und viele Materialeigenschaften des Implantats in jeder Raumrichtung gemäß einer stetigen Funktion veränderlich gestaltet werden können.

Die Erfindung bezieht sich außer auf ein Verfahren der oben beschriebenen Art auch auf ein Implantat, dass hergestellt nach einem Verfahren der oben beschriebenen Art hergestellt ist.

Im Folgenden wird die Erfindung in Figuren einer Zeichnung gezeigt und nachfolgend erläutert.

### Dabei zeigt

- Figur 1:: schematisch das additive Fertigungsverfahren für ein Implantat, das aus mehreren Materialien gefertigt wird,
- Figur 2:: eine vereinfachte Gesamtansicht eines Knochenimplantats,
- Figur 3:: einen Körper mit einem schräg gegenüber einer z-Achse des Schichtauftrags verlaufenden Verbindungsbereich,
- Figuren 4, 5:: den Verlauf des Schichtaufbaus für eine erste Variante des Verfahrens,
- Figuren 6, 7:: den Verlauf des Schichtaufbaus für eine zweite Variante des Verfahrens,
- Figur 8:: einen Körper mit einem Aufbau von vollständigen Schichten, sowie
- Figur 9:: einen Schichtaufbau in einem Verbindungsbereich im Detail.

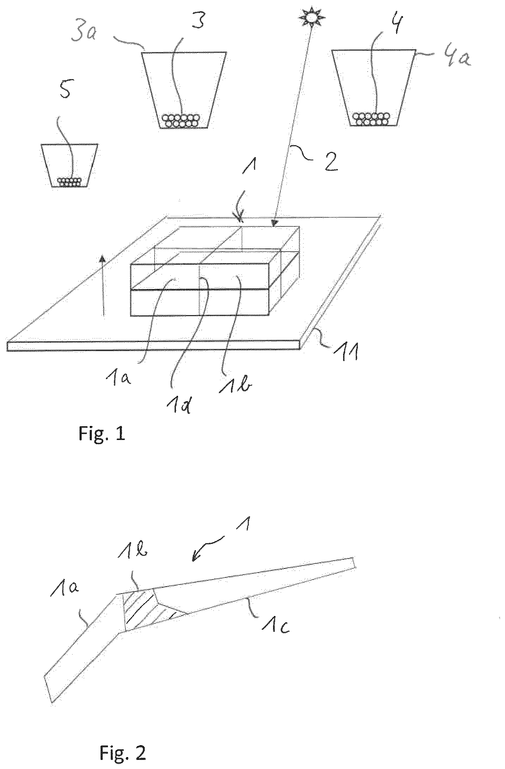

Figur 1 zeigt in einer perspektivischen Darstellung den Ablauf eines additiven Fertigungsverfahrens, konkret eines Pulverbett- Laserschmelzverfahrens. Ein Körper, der zu einem Implantat 1 aufgebaut wird, wird schichtweise in der Form von Pulverschichten auf einer Plattform 11 aufgebaut. Der Körper und später das Implantat ist aus mehreren Bauteilabschnitten 1a, 1b zusammengesetzt, die in einem Bereich 1d aneinander angrenzen und stoffschlüssig miteinander verbunden sind. Dazu werden in den einzelnen Bauteilabschnitten 1a, 1b nacheinander Pulverschichten von Pulvern abgelegt, die aus verschiedenen Materialien bestehen. Beispielsweise kann in dem gezeigten Beispiel der erste Bauteilabschnitt 1a, der durch einen Quader in der vorderen oberen linken Ecke des Körpers 1 gebildet ist, aus einem Tantalpulver aufgeschichtet werden, während der zweite Bauteilabschnitt 1b, der in der Figur 1 im vorderen, oberen rechten Teil des Körpers 1 liegt, aus einem Titanpulvermaterial, beispielsweise einer Titanlegierung, insbesondere einem Ti6Al4V- Pulver aufgeschichtet werden kann. In der Figur 1 sind symbolisch für den unterschiedlichen Materialauftrag der Behälter 3a mit dem Pulvermaterial 3 und der Behälter 4a mit dem Pulvermaterial 4 dargestellt. In bekannter Weise wird das Pulver nach dem Schichtauftrag jeweils schichtweise selektiv und ortsabhängig verfestigt, um einen 3-dimensionalen Körper in gewünschter Form herzustellen. Die Verfestigung kann grundsätzlich mit einem Binderverfahren oder mit einem Energiestrahl stattfinden. Im Binderverfahren wird selektiv, beispielsweise in einem Druckverfahren, ein Bindermaterial hinzugefügt, das abbindet und das Pulver verfestigt. Energiestrahlen können als Strahlung, beispielsweise in Form eines Lasers, oder als Teilchenstrahlen, beispielsweise in Form eines Elektronenstrahls ausgeführt sein, wobei andere Arten von Energiestrahlung ebenfalls denkbar sind, wie beispielsweise lonenstrahlen. Der Energieeintrag der jeweiligen Strahlen bewirkt eine Temperaturerhöhung in dem jeweiligen Pulver bis zu einer Versinterung oder einem, wenigstens teilweisen Aufschmelzen des Materials. Beim Abkühlen hat sich der bestrahlte Bereich dann verfestigt. Auf diese Weise können auch verschiedene Materialien, beispielsweise ein Titanwerkstoff und ein Tantalwerkstoff, in dem Bereich, in dem die Bauteilabschnitte aneinander angrenzen, stoffschlüssig miteinander verbunden werden. Dabei kann mit dem additiven Fertigungsverfahren die Materialgrenze sehr frei gestaltet werden. Es kann damit ein Implantat gebildet werden, das wenigstens einen aus einem Titanmaterial und einem aus einem Tantalmaterial bestehenden Bauteilabschnitt aufweist.

Tantal ist deutlich biokopatibler als Titan, während Titan inerter ist und eine höhere mechanische Festigkeit als Tantal aufweist.

In der Kombination der beiden Werkstoffe kann somit ein Implantat gebildet werden, dessen einer Teil, bevorzugt aus dem Tantalmaterial, zum besonders guten Einwachsen in einen Knochen vorgesehen ist, während andere Bereiche, insbesondere aus einem Titanmaterial, im Implantierten Zustand aus einem Knochen herausragen können und dazu geeignet sind, große Kräfte, insbesondere Dauerwechselbelastungen, dauerhaft zu übertragen. Insbesondere der aus Tantalmaterial bestehende Bauteilabschnitt kann auch porös/schwammartig gestaltet sein, um das Einwachsen von Gewebe zusätzlich zu fördern.

Ein derartiges Implantat ist in der Figur 2 dargestellt, wobei dieses die Bauteilabschnitte 1a, 1b, 1c aus verschiedenen Materialien aufweist. Die Bauteilabschnitte 1a, 1c bestehen beispielsweise aus einem Titanmaterial, während der zwischen ihnen liegende Bauteilabschnitt ab aus einem schwammartigen, porösen Tantalmaterial besteht. Durch die schwammartige Gestaltung wird mittels der Oberflächenstruktur das Einwachsen von Gewebe begünstigt. Der Bereich 1d, in dem der erste und der zweite Bauteilabschitt 1a, 1b aneinander angrenzen, kann als gemeinsame Kontaktfläche mit stoffschlüssiger Fügung gestaltet sein, jedoch in vielen Fällen vorteilhafter als ein Verbindungsbereich, in dem die beiden Materialien, aus denen der erste und der zweite Bauteilabschnitt bestehen, also beispielsweise ein Titan- und ein Tantalmaterial, in einem bestimmten Maß miteinander vermischt sind.

Dies kann dadurch gelingen, dass in dem Verbindungsbereich durch das mehr oder weniger starke Aufschmelzen der Pulverwerkstoffe beide Materialien verflüssigt werden und sich punktuell vermischen. Die Vermischung kann dadurch unterstützt und der Verbindungsbereich vergrößert werden, dass in diesem Bereich die Schichten aus den verschiedenen Materialien miteinander verzahnt werden und/ oder im Verbindungsabschnitt von vornherein ein Pulver verwendet wird, das eine Mischung der Materialien des ersten und zweiten Bauteilabschnitts darstellt.

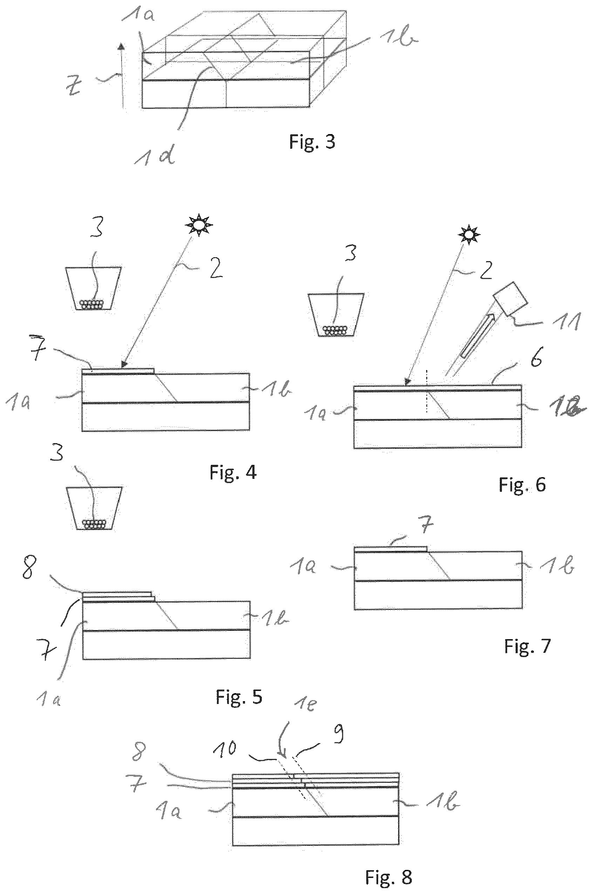

Die erstgenannte Variante der Verzahnung von Schichten wird weiter unten anhand der Figuren 4 bis 9 noch näher erläutert.

Die zweite Variante sieht außer dem ersten und dem zweiten Pulvermaterial 3. 4 noch ein drittes Pulvermaterial 5 aus einem entsprechenden Behälter 5a mit einer entsprechenden Zuführung zur Arbeitsplattform 11 vor, das eine Mischung mit dem ersten und dem zweiten Material darstellt. Es können zur Schaffung eines allmählichen Materialüberganges in dem herzustellenden Körper auch mehrere dritte Pulvermaterialien mit verschiedenen Mischungsverhältnissen des ersten und zweiten Materials verendet werden, um noch feiner gradierte Übergänge im Verlauf des Verbindungsabschnittes zu erzeugen.

Die Zuführungen des Pulvermaterialien von den verschiedenen Vorratsbehältern 3a, 4a, 5a zur Arbeitsplattform 11 sind in den Figuren der Übersichtlichkeit halber nicht dargestellt, jedoch sind verschiedene Konstruktionen im Stand der Technik bekannt.

Die verschiedenen pulverförmigen Materialien können in an sich bekannter Weise zu der Arbeitsplattform der additiven Fertigungseinrichtung transportiert und beispielsweise mittels Düsen oder Kanälen mit Luftstromunterstützung oder durch Vibration abgelegt werden. Jede horizontale Schicht des herzustellenden Körpers kann auf diese Weise aus drei oder mehr verschiedenen Materialien mosaikartig zusammengesetzt werden, wobei auf mikroskopischer Ebene durch das Aufschmelzen in den Grenzbereichen zwischen den verschiedenen Materialien noch eine weitere Vermischung und auch Legierungsbildung stattfinden kann.

Dabei kann der Energieeintrag durch geeignete Wahl der Strahlparameter und der Strahlführungsparameter des Energiestrahls, insbesondere Laserstrahls so gewählt werden, dass jedes der beiden Materialien des ersten und zweiten Bauteilabschnittes entweder gesintert oder voll aufgeschmolzen wird und sich verfestigt. Wird für den Verbindungsabschnitt eine Temperatur gewählt, die zwischen den Temperaturen liegt, die jeweils für den ersten und zweiten Bauteilabschnitt gewählt wurden, so wird in den meisten Fällen die Pulververfestigung in diesem Bereich nach ähnlichem Muster erfolgen, wie in den beiden Bauteilabschnitten.

Wird die Zieltemperatur im Verbindungsabschnitt durch den Energiestrahl höher eingestellt als für den ersten und zweiten Bauteilabschnitt, so kann ein stärkeres Aufschmelzen und Verflüssigen des Materials im Verbindungsabschnitt erfolgen, wodurch eine stärkere Durchmischung, eine intensivere Verbindung und in vielen Fällen eine Legierungsbildung in diesem Bereich erreicht werden kann.

Die Figur 3 zeigt einen Körper, der verschiedene Bauteilabschnitte 1a, 1b aufweist, die in einem Bereich 1d aneinander angrenzen. Der Bereich 1d ist als schrägliegende Fläche dargestellt, die etwa in einem Winkel von 45 Grad gegenüber der Schichtungsrichtung z der additiven Fertigungseinrichtung geneigt ist. Es soll beispielhaft anhand der Figuren 4 bis 9 gezeigt werden, wie auch ein derart orientierter Grenzbereich in dem additiven Fertigungsverfahren mit zwei oder mehr Materialien im Pulverbett-Laserschmelzverfahren gestaltet werden kann.

In der Figur 4 ist in einer ersten Variante dargestellt, dass zur Formung des schrägen Grenzbereichs 1d in einem Maskierverfahren ein Pulver 3 eines ersten Materials, beispielsweise eines Tantalmaterials, nur in dem Bereich der Bauteiloberfläche in Form der Schicht 7 aufgetragen wird, wo der erste Bauteilabschnitt 1a fortgesetzt/weiter aufgebaut wird. Danach wird dieser Bereich mit einem Laserstrahl verfestigt und es wird eine weitere Schicht aufgetragen, entweder auf die erste Schicht 7 oder mit einem anderen Pulvermaterial auf den Teil der Bauteiloberfläche, auf dem der zweite Bauteilabschnitt 1b fortgesetzt/weiter aufgebaut wird.

In der Figur 5 ist dargestellt, dass eine weitere Schicht 8 auf die erste Schicht 7 aufgebracht und dort verfestigt wird. Die Schicht 8 trägt mit ihrer geometrischen Form und Begrenzung zur Ausbildung des Grenzbereiches 1b in schräger Richtung zur z- Richtung bei.

In den Figuren 6 und 7 ist ein zweites mögliches Vorgehen bei der Materialschichtung gezeigt. In der Figur 6 ist dargestellt, dass die gesamte Bauteiloberfläche mit einem Pulvermaterial 3 abgedeckt wird. Das Pulvermaterial wird jedoch nur in dem Bereich mit einem Laserstrahl 2 verfestigt, in dem der erste Bauteilabschnitt 1a fortgesetzt werden soll. Der nicht verfestigte Teil des Materials kann entfernt, beispielsweise mittels einer Saugvorrichtung 11 abgesaugt werden. Danach wird die nächste Schicht eines Pulvermaterials aufgetragen und selektiv verfestigt. In dem Beispiel der Figur 7 ist sichtbar, dass die Schicht 7 verfestigt ist und den ersten Bauteilabschnitt fortsetzt. Im nächsten Schritt könnte auch nach dieser Methode die Schicht 8 zur weiteren Fortsetzung des ersten Bauteilabschnitts 1a aus einem Tantalmaterial aufgebracht werden, indem die nächste Pulverschicht vollflächig aufgebracht und nur im Bereich 8 verfestigt wird. Alternativ kann auch vollflächig eine Schicht aus dem zweiten Pulvermaterial, beispielsweise Titan, aufgebracht und nur im rechten Bereich der Bauteiloberfläche zur Fortsetzung des zweiten Bauteilabschnitts 1b verfestigt werden. Im linken Bereich der Bauteiloberfläche über der Schicht 7 würde danach das überschüssige Pulver abgesaugt.

Im Ergebnis entsteht dabei ein Schichtkörper, wie er in der Figur 8 dargestellt ist, wobei der Übergang/Grenzbereich zwischen den beiden Materialien sich von Schicht zu Schicht 7, 8 horizontal verschiebt, so dass die Grenzfläche oder ein sich ausbildender Verbindungsabschnitt 1e im Querschnittsbild zwischen den Linien 9 und 10 schräg zur z- Achse, der Achse der Materialschichtung, verläuft.

Wie bereits oben erwähnt, können die beiden Materialien in diesem Verbindungsabschnitt durch einen Energiestrahl auf eine besonders hohe Temperatur erhitzt werden, die höher ist als die Temperaturen, bei denen die beiden Materialien in dem ersten und zweiten Bauteilabschnitt verfestigt werden. Alternativ oder zusätzlich kann in dem Verbindungsabschnitt 1e auch die Verbindungstemperatur länger gehalten werden als in den beiden Bauteilabschnitten 1a, 1b.

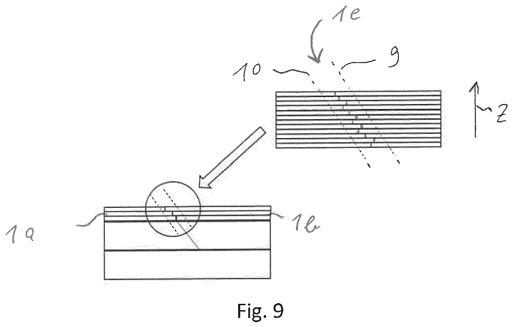

Eine weitere Maßnahme zu einer Vermischung der beiden Materialien des ersten und zweiten Bauteilabschnitts kann vorsehen, dass die Schichten der verschiedenen Materialien im Verbindungsabschnitt derart miteinander verzahnt sind, dass eine oder mehrere Schichten des ersten Materials jeweils ein Stück weit in einen Bereich zwischen jeweils zwei Schichten des anderen Bereichs hineinragt/hineinragen. Dies ist in der Figur 9 in dem rechten Bereich dargestellt, in dem der eingekreiste Bereich der unteren Darstellung vergrößert gezeigt ist. Die Grenze zwischen den beiden Materialien der Bauteilabschnitte 1a, 1b springt innerhalb des Verbindungsabschnitts 1e zwischen den Linien 9 und 10 von Schicht zu Schicht nach links oder rechts, wodurch sich die genannte Verzahnung ergibt. Zudem kann auch vorgesehen sein, dass im Bereich des Verbindungsabschnitts jeweils geringere Schichtdicken aufgebracht und verfestigt werden, als im ersten und zweiten Bauteilabschnitt, um eine bessere Vermischung der Materialien, wenn gewünscht auch in gradiertem Profil, zu erreichen.

## Patentansprüche

1. Verfahren zur Herstellung eines Implantats (1), wobei das Implantat wenigstens zwei miteinander verbundene Bauteilabschnitte (1a, 1b, 1c) aufweist, die wenigstens in einem Bereich (1d), in dem sie aneinander angrenzen, durch ein additives Fertigungsverfahren in Form eines Pulverbett-Verbindungsprozesses hergestellt werden, wobei ein erster Bauteilabschnitt (1a) aus einem ersten Material und ein zweiter Bauteilabschnitt (1b) aus einem zweiten Material hergestellt wird, wobei das erste und das zweite Material sich durch das Maß ihrer Bio-Kompatibilität unterscheiden.

2. Verfahren nach Anspruch 1, **dadurch gekennzeichnet, dass** das erste und das zweite Material sich durch das Maß ihrer Bio-Inertheit unterscheiden.

3. Verfahren nach einem der Ansprüche 1 oder 2, **dadurch gekennzeichnet, dass** das erste und/oder das zweite Material ein Metall ist.

4. Verfahren nach Anspruch 1,2 oder 3, **dadurch gekennzeichnet, dass** das erste oder das zweite Material Tantal oder eine Tantal-Legierung ist.

5. Verfahren nach Anspruch 1, 2, 3 oder 4, **dadurch gekennzeichnet, dass** das erste oder das zweite Material Titan oder eine Titan-Legierung, insbesondere die Titanlegierung Ti6Al4V ist.

6. Verfahren nach einem der Ansprüche 1, bis 5, **dadurch gekennzeichnet, dass** zwischen dem ersten Bauteilabschnitt (1a) und dem zweiten Bauteilabschnitt (1b) ein Verbindungsabschnitt (1e) gebildet wird, der aus einer Mischung wenigstens des ersten Materials und des zweiten Materials, insbesondere, falls sowohl das erste als auch das zweite Material ein Metall ist, wenigstens teilweise aus einer Legierung mit dem ersten und dem zweiten Material besteht.

7. Verfahren nach Anspruch 6, **dadurch gekennzeichnet, dass** der Verbindungsabschnitt mit einer gradierten Zusammensetzung gebildet wird, die in einer oder mehreren Richtungen ortsabhängig variierende Anteile des ersten und des zweiten Materials aufweist.

8. Verfahren nach einem der Ansprüche 1 bis 7, **dadurch gekennzeichnet, dass** der erste Bauteilabschnitt (1a) und der zweite Bauteilabschnitt (1b) zur Verfestigung jeweils mit einem Energiestrahl (2) bestrahlt wird, wobei durch die Wahl unterschiedlicher Strahlparameter und/oder Strahlführungsparameter in den beiden Bauteilabschnitten verschiedene lokale Verbindungstemperaturen erreicht werden.

9. Verfahren nach Anspruch 8, **dadurch gekennzeichnet, dass** in dem Verbindungsabschnitt (1e) durch die Wahl der Strahlparameter und/oder Strahlführungsparameter eine Verbindungstemperatur erreicht wird, die zwischen den Verbindungstemperaturen des ersten und des zweiten Bauteilabschnitts (1a, 1b) oder die oberhalb der höheren der Verbindungstemperaturen des ersten und des zweiten Bauteilabschnitts liegt.

10. Verfahren nach Anspruch 8 oder 9, **dadurch gekennzeichnet, dass** der Energiestrahl (2) zum Verfestigen der Materialien als Laserstrahl oder als Elektronenstrahl ausgebildet ist.

11. Verfahren nach einem der Ansprüche 1 bis 10, **dadurch gekennzeichnet, dass** in dem Verbindungsabschnitt (1e) jeweils in einem Schritt ausschließlich Pulvermengen (3, 4) abgelegt werden, die entweder aus dem ersten Material oder aus dem zweiten Material oder aus einem dritten Material bestehen oder dass in einem Schritt zumindest Bereichsweise im Verbindungsbereich eine Pulvermischung (5) abgelegt wird, die das erste und das zweite Material oder das erste und das zweite Material sowie ein drittes Material enthält.

12. Verfahren nach Anspruch 11, **dadurch gekennzeichnet, dass** jede Pulverschicht (6) des ersten und zweiten Materials jeweils beide Bauteilabschnitte und insbesondere den Verbindungsabschnitt vollflächig abdeckend abgelegt wird und dass nicht verfestigte Bereiche des aufgetragenen Materials nach der Verfestigung von Teilen der jeweiligen Schicht abgetragen werden.

13. Verfahren nach Anspruch 11, **dadurch gekennzeichnet, dass** eine oder mehrere oder alle Pulverschichten (7, 8) des ersten und zweiten Materials jeweils nur in den Bereichen der Bauteilabschnitte (1a, 1b) und des Verbindungsabschnitts abgelegt werden, an denen sie verfestigt werden.

14. Verfahren nach einem der Ansprüche 1 bis 13, **dadurch gekennzeichnet, dass** wenigstens ein Verbindungsbereich (1e) sich durch wenigstens zwei, insbesondere wenigstens drei, weiter insbesondere wenigstens zehn Schichten erstreckt, wobei weiter insbesondere wenigstens eine Grenze (9, 19) des Verbindungsbereiches schräg durch die Schichten und/oder dass der erste und/oder der zweite Bauteilabschnitt (1a, 1b) und/oder der Verbindungsabschnitt einen oder mehrere Hohlräume aufweist und insbesondere eine schwammartige Struktur aufweist.

15. Implantat, **dadurch gekennzeichnet, dass** es nach einem Verfahren gemäß einem der Ansprüche 1 bis 14 hergestellt ist.
